# EUROPÄISCHE PATENTSCHRIFT

(11) **EP 1 185 175 B1**
(45) Veröffentlichungstag und Bekanntmachung des Hinweises auf die Patenterteilung: **23.04.2003**
(21) Anmeldenummer: 00936870.5
(22) Anmeldetag: 07.06.2000
(51) Int. Cl.: A01N 37/42

(54) **VERFAHREN ZUR ERHÖHUNG VOM GEHALT AN FLAVONOIDEN UND PHENOLISCHEN INHALTSSTOFFEN IN PFLANZEN**
METHOD OF INCREASING THE CONTENT OF FLAVONOIDS AND PHENOLIC SUBSTANCES IN PLANTS
PROCEDE PERMETTANT D'AUGMENTER LA TENEUR EN FLAVONOIDES ET EN COMPOSANTS PHENOLIQUES DANS DES PLANTES

(30) Priorität: 17.06.1999 DE 19927571
(43) Veröffentlichungstag der Anmeldung: 13.03.2002
(73) Patentinhaber: BASF AKTIENGESELLSCHAFT, 67056 Ludwigshafen (DE)
(72) Erfinder: RADEMACHER, Wilhelm, D-67117 Limburgerhof (DE); KRÄMER, Klaus, D-76829 Landau (DE); SCHWEDEN, Jürgen, D-67433 Neustadt (DE)
(86) Internationale Anmeldenummer: EP0005258
(87) Internationale Veröffentlichungsnummer: WO00078143

(56) Entgegenhaltungen:
- EP-A- 0 598 404
- FR-A- 2 767 533
- ROEMMELT, S. ET AL: "Effects of prohexadione-Ca on the flavonoid metabolism of apple respect to plant resistance against fire blight" ACTA HORTIC. (1999), 489(EIGHTH INTERNATIONAL WORKSHOP ON FIRE BLIGHT, 19, Seiten 359-363, XP000917326 in der Anmeldung erwähnt
- RADEMACHER, WILHELM: "Inhibitors of gibberellin biosynthesis: Prohexadione-Ca, a new plant grow regulator for apple with interesting biochemical features" RIKEN REV., Nr. 21, April 1999 (1999-04), Seiten 11-12, XP000917297

## Beschreibung

Gegenstand der vorliegenden Erfindung ist ein Verfahren zur Erhöhung des Gehalts an Flavonoiden und phenolischen Inhaltsstoffen in Pflanzen, dadurch gekennzeichnet, daß die Pflanze mit wachstumsregulierenden Acylcyclohexadionen der Formel I behandelt werden, 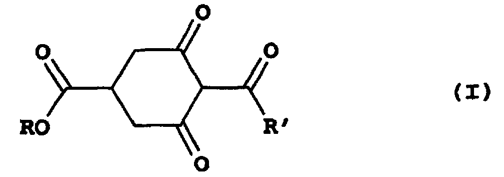 wobei R insbesondere für Wasserstoff, eine C₁-C₆-Alkyl-, C₁-C₆-Haloalkyl-, C₂-C₁₀-Alkylthioalkyl- oder Phenylgruppe (substituiert oder unsubstituiert) und R' für Wasserstoff, eine C1-C6-Alkyl-, C3-C6-Cycloalkyl-, Benzyl- (substituiert oder unsubstituiert), Phenylethyl-, Phenoxyethyl-, 2-Thienylmethyl-, Alkoxymethyl- oder Alkylthiomethylgruppe steht sowie geeignete Salze dieser Verbindungen.

Besonders bevorzugt ist ein Verfahren, bei dem die Erhöhung durch Behandlung mit Acylcyclohexadionen wie Prohexadion-Ca(II) und/oder Trinexapac-ethyl(III) hervorgerufen werden.

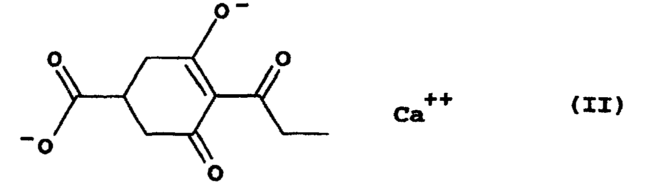

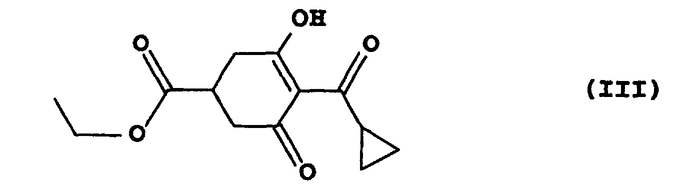

Weiterer Gegenstand der Erfindung ist die Verwendung von Pflanzen, die nach dem erfindungsgemäßen Verfahren mit Acylcyclohexadionen der Formel I, speziell Prohexadion-Ca oder mit Trinexapac-ethyl behandelt wurden oder von Teilen dieser Pflanzen oder aus ihnen hergestellte Produkte (Säfte, Tees, Extrakte, Fermentationsprodukte und -rückstände) zur Herstellung von heilenden, gesundheitsfördernden oder stärkenden Mitteln für Mensch und Tier sowie von Kosmetika.

Weiterer Gegenstand der Erfindung sind Mittel, hergestellt nach den erfindungsgemäßen Verfahren, dadurch gekennzeichnet, daß Trauben von roten Weinreben gewonnen und verarbeitet werden, deren Anthocyanbildung durch Behandlung mit Acylcyclohexadionen wie Prohexadion-Ca oder Trinexapac-ethyl ganz oder teilweise unterbunden worden ist und die sich daher durch einen qualitativ und quantitativ erhöhten Gehalt an Flavonoiden und anderen phenolischen Inhaltsstoffen auszeichnen.

Verschiedene phenolische Substanzen (Phenylpropanoide) kommen in Pflanzen vor, z. B. Kaffeesäure, Ferulasäure, Chlorogensäure, Gallussäure, Eugenol, Lignane, Cumarine, Lignin, Stilbene (Polydatin, Resveratrol), Flavonoide (Flavone, Catechine, Flavanone, Anthocyanidine, Isoflavone), polymethoxylierte Flavone. Demgemäß sind Phenole auch genereller Bestandteil vieler pflanzlicher Nahrungs- und Genußmittel. Bestimmte phenolische Substanzen sind von besonderer Bedeutung, da sie nach Aufnahme mit der Nahrung im menschlichen oder tierischen Stoffwechsel eine antioxidative Wirkung ausüben können (Baum, B. O,; Perun, A. L. Antioxidant efficiency versus structure. *Soc. Plast. Engrs Trans* **2**: 250-257, (1962); Gardner, P.T.; McPhail, D.B.; Duthie, G.G. Electron spin resonance spectroscopic assessment of the antioxidant potential of teas in aqueous and organic media. *J. Sci. Food Agric.* **76**: 257-262, (1997); Rice-Evans, C. A.; Miller, N. J.; Pananga, G. Structure-antioxidant activity relationship of flavonoids and phenolic acids. *Free Radic. Biol. Med.* **20:** 933-956, (1996); Salah, N.; Miller, N. J.; Paganga, G.; Tijburg, L.; Bolwell, G. P.; Rice-Evans, C. Polyphenolic flavonoids as scavenger of aqueous phase radicals and as chain-breaking antioxidants. *Arch Biochem Biophys* **322:** 339-346, (1995); Stryer, *L. Biochemistry S.* Francisco: Freeman, (1975); Vieira, O.; Escargueil-Blanc, I.; Meilhac, O.; Basile, J. P.; Laranjinha, J.; Almeida, L.; Salvayre, R.; Negre-Salvayre, A. Effect of dietary phenolic compounds on apoptosis of human cultured endothelial cells induced by oxidized LDL. *Br J Pharmacol* **123:** 565-573, (1998)). Darüber hinaus haben Polyphenole vielfältige Wirkungen auf den Zellstoffwechsel. Unter anderem werden Enzyme der Signaltransduction wie Proteinkinase C, Tyrosin-Proteinkinase und Phosphatidylinositol-3-kinase moduliert (Agullo, G.; Gamet-payrastre, L.; Manenti, S.; Viala, C.; Remesy, C.; Chap, H.; Payrastre, B. Relationship between flavonoid structure and inhibition of phosphatidylinositol 3-kinase: a comparison with tyrosine kinase and protein kinase C inhibition. *Biochem Pharmacol* **53** :1649-1657, (1997); Ferriola, P. C.; Cody, V.; Middleton, E. Protein kinase C inhibition by plant flavonoids. Kinetic mechanisms and structure activity relationship. *Biochem Pharmacol* **38:** 1617-1624, (1989); Cushman, M.; Nagarathman, D.; Burg, D. L.; Geahlen, R. L. Synthesis and protein-tyrosine kinase inhibitory activity of flavonoids analogues. *J Meed Chem* **34***:* 798-806, (1991); Hagiwara, M.; Inoue, S.; Tanaka, T.; Nunoki. K.; Ito, M.; Hidaka, H. Differential effects of flavonoids as inhibitors of tyrosine protein kinases and serine/threonin protein kinases. *Biochem Pharmacol* **37*:*** 2987-2992, (1988)), -die induzierbare NO-Synthase downreguliert (Kobuchi, H.; Droy-Lefaix, M. T.; Christen, Y.; Packer, L. *Ginkgo biloba* extract (EGb761): inhibitory effect on nitric oxide production in the macrophage cell line RAW 264.7. *Biochem Pharmacol* **53:** 897-903, (1997)) und die Genexpression von z. B. Interleukinen und Adhäsionsmolekülen (ICAM-1, VCAM-1) reguliert (Kobuchi, H.; Droy-Lefaix, M. T.; Christen, Y.; Packer, L. *Ginkgo biloba* extract (EGb761): inhibitory effect on nitric oxide production in the macrophage cell line RAW 264.7. *Biochem Pharmacol* **53:**897-903, (1997); Wolle, J.; Hill, R. R.; Ferguson, E.; Devall, L. J.; Trivedi, B. K.; Newton, R. S.; Saxena, U. Selective inhibition of Tumor necrosis Factorinduced vascular cell adhesion molecule-1 gene expression by a novel flavonoid. Lack of effect on transcriptional factor NF-kB. *Atherioscler Thromb Vasc Biol* **16:** 1501-1508, (1996)). Es gilt als gesichert, daß diese Wirkungen positiv sind zur Prävention von Herz-Kreislauferkrankungen, Diabetes, verschiedener Tumorarten und weiterer chronischer Krankheiten (Bertuglia, S.; Malandrino, S.; Colantuoni, A. Effects of the natural flavonoid delphinidin on diabetic microangiopathy. *Arznei-Forsch*/*Drug Res* **45:** 481-485, (1995); Griffiths, K.; Adlercreutz, H.; Boyle, P.; Denis, L.; Nicholson, R.I.; Morton, M.S. *Nutrition and Cancer* Oxford: Isis Medical Media, (1996); Hertog, M. G. L.; Fesrens, E. J. M.; Hollman, P. C. K.; Katan, M. B.; Kromhout, D. Dietary antioxidant flavonoids and risk of coronary heart disease: the Zutphen elderly study. *The Lancet* **342:** 1007-1011, (1993); Kapiotis, S.; Hermann, M.; Held, I.; Seelos, C.; Ehringer, H.; Gmeiner, B. M. Genistein, the dietary-derived angiogenesis inhibitor, prevents LDL oxidation and protects endothelial cells from damage by atherogenic LDL. *Arterioscler Thromb Vasc Biol* **17**: 2868-74, (1997); Stampfer, M. J.; Hennekens, C. H.; Manson, J. E.; Colditz, G. A.; Rosner, B.; Willet, W. C. Vitamin E consumption and the risk of coronary disease in women. *New Engl J Med* **328 :**1444-1449, (1993); Tijburg, L. B. M.; Mattern, T.; Folts, J. D.; Weisgerber, U. M.; Katan, M. B. Tea flavonoids and cardiovascular diseases: a review. *Crit Rev Food Sci Nutr* **37:** 771-785, (1997); Kirk, E. A.; Sutherland, P.; Wang, S. A.; Chait, A.; LeBoeuf, R. C. Dietary isoflavones reduce plasma cholesterol and atherosclerosis in C57BL/6 mice but not LDL receptor-deficient mice. *J Nutr* **128:** 954-9, (1998)). Aus geeigneten Pflanzen wird daher bereits eine Reihe von heilenden, gesundheitsfördernden oder stärkenden Mitteln gewonnen, deren Wirkung auf ihrem Gehalt an phenolischen Substanzen beruht (Gerritsen, M. E.; Carley, W. W.; Ranges, G. E.; Shen, C. P.; Phan, S. A.; Ligon, G. F.; Perry, C. A. Flavonoids inhibit cytokine-induced endothelial cell adhesion protein gene expression. *Am J Pathol* **147:** 278-292, (1995); Lin, J. K.; Chen, Y. C.; Huang, Y. T.; Lin-Shiau, S. Y. Suppression of protein kinase C and nuclear oncogene expression as possible molecular mechanisms of cancer chemoprevention by apigenin and curcumin. *J Cell Biochem Suppl* **28-29:**39-48, 1997; Zi, X.; Mukhtar, H.; Agarval, R. Novel cancer chemoprevenctive effects of a flavonoid antioxidant silymarin: inhibition of mRNA expression of an endogenous tumor promoter TNF alpha. *Biochem Biophys Res Comm* **239:**334-339, 1997). Bekannt ist weiterhin, daß bestimmte pflanzliche Nahrungsmittel oder aus ihnen hergestellte Genußmittel eine positive Wirkung gegen verschiedene Krankheiten ausüben. Das in Weißwein, besonders aber in Rotwein, enthaltene Resveratrol (nebst weiterer Komponenten) wirkt beispielsweise gegen kardiovasculäre Erkrankungen und Krebs (Gehm, B.D.; McAndrews, J.M.; Chien, P.-Y.; Jameson, J.L. Resveratrol, a polyphenolic compound found in grapes and wine, is an agonist for estrogen receptor. *Proc Natl Acad Sci USA* **94:** 14138-14143, (1997); Jang, M.; Cai, L.; Udeani, G.O.; Slowing, K.V.; Thomas, C.F.; Beecher, C.W.W.; Fong, H.H.S; Farnsworth, N.R.; Kinghorn, A.D.; Mehtha, R.G.; Moon, R.C., Pezzuto, J.M. Cancer chemopreventive activity of resveratrol, a natural product derived from grapes. *Science* **275:** 218-220, (1997)). Eine ähnliche Wirkung weisen auch Substanzen wie Catechin, Epicatechin-3-gallat, Epigallocatechin und Epigallocatechin-3-gallat auf, die in Blättern von Tee (*Camellia sinensis*) vorkommen. Insbesondere aus nicht-fermentierten Teeblättern (Grüner Tee) hergestellte Getränke sind von positiver gesundheitlicher Relevanz (Hu, G.; Han, C.; Chen, J. Inhibition of oncogene expression by green tea and (-)-epigallocatechin gallate in mice. *Nutr Cancer* **24:** 203-209; (1995); Scholz, E; Bertram, B. *Camellia sinensis* (L.) O. Kuntze. Der Teestrauch. *Z. Phytotherapie* ***17:*** *235-250*, (1995); Yu, R.; Jiao, J. J.; Duh, J. L.; Gudehithlu, K.; Tan, T. H.; Kong, A. N. Activation of mitogen-activated protein kinases by green tea polyphenols: potential signaling pathways in the regulation of antioxidant responsive elements-mediated phase II enzyme gene expression. *Carcinigenesis* **18:** 451-456, (1997); Jankun, J.; Selman, S.H.; Swiercz, R. Why drinking green tea could prevent cancer. *Nature* **387:** *561,* (1997)). Darüber hinaus weisen auch polymethoxylierte Flavone aus Zitrusfrüchten eine potentielle antitumorale Wirkung auf (Chem, J.; Montanari, A.M.; Widmer, W.W. Two new polymethoxylierte flavone, a class of compounds with potential anticancer activity, isolated from cold pressed dancy tangerine peel oil solids. J

*Agric Food Chem* ***45:*** *364-368,* (1997)).

Acylcyclohexadione wie Prohexadion-Ca und Trinexapac-ethyl (ältere Bezeichnung: Cimectacarb) werden als Bioregulatoren zur Hemmung des pflanzlichen Längenwachstums eingesetzt. Ihre bioregulatorische Wirkung kommt dadurch zustande, daß sie die Biosynthese von längenwachstumsfördernden Gibberellinen blockieren. Dabei hemmen sie aufgrund ihrer strukturellen Verwandtschaft zu 2-Oxoglutarsäure bestimmte Dioxygenasen, die 2-Oxoglutarsäure als Co-Substrat benötigen (Rademacher, W, Biochemical effects of plant growth retardants, in: Plant Biochemical Regulators, Gausman, HW (ed.), Marcel Dekker, Inc., New York, pp. 169-200 (1991)). Es ist bekannt, daß derartige Verbindungen auch in den Stoffwechsel von Phenolen eingreifen und so bei mehreren Pflanzenarten eine Hemmung der Anthocyanbildung bewirken können (Rademacher, W et al., The mode of action of acylcyclohexanediones - a new type of growth retardant, in: Progress in Plant Growth Regulation, Karssen, CM, van Loon, LC, Vreugdenhil, D (eds.), Kluwer Academic Publishers, Dordrecht (1992)). Derartige Effekte auf den Haushalt phenolischer Inhaltsstoffe werden als ursächlich für die Nebenwirkung von Prohexadion-Ca gegen Feuerbrand angegeben (Rademacher, W et al., Prohexadione-Ca - a new plant growth regulator for apple with interesting biochemical features, Poster auf dem 25^{th} Annual Meeting of the Plant Growth Regulation Society of America, 7.-10. Juli 1998, Chicago). A. Lux-Endrich (Dissertation Technische Universität München in Weihenstephan, 1998) findet im Verlauf ihrer Untersuchungen zum Wirkmechanismus von Prohexadion-Ca gegen Feuerbrand, daß es in Zellkulturen von Apfel durch Prohexadion-Ca zu einer mehrfachen Erhöhung des Gehaltes an phenolischen Substanzen kommt und daß dabei eine Reihe von sonst nicht vorhandenen Phenolen auftritt. Im Rahmen dieser Untersuchungen wurde weiterhin gefunden, daß unter dem Einfluß von Prohexadion-Ca relativ hohe Mengen von Luteoliflavan und Eriodyctiol in Sproßgewebe von Apfel auftreten. Luteoliflavan kommt in Apfelgewebe normalerweise nicht vor und Eriodyctiol tritt als Intermediat des Flavonoidstoffwechsels nur in geringen Mengen auf. Die zu erwartenden Flavonoide Catechin und Cyanidin waren im behandelten Gewebe jedoch nicht nachweisbar oder traten nur in deutlich reduzierten Mengen auf (S. Römmelt et al, Vortrag 8^{th} International Workshop on Fire Blight, Kusadasi, Türkei, 12.-15. Oktober 1998).

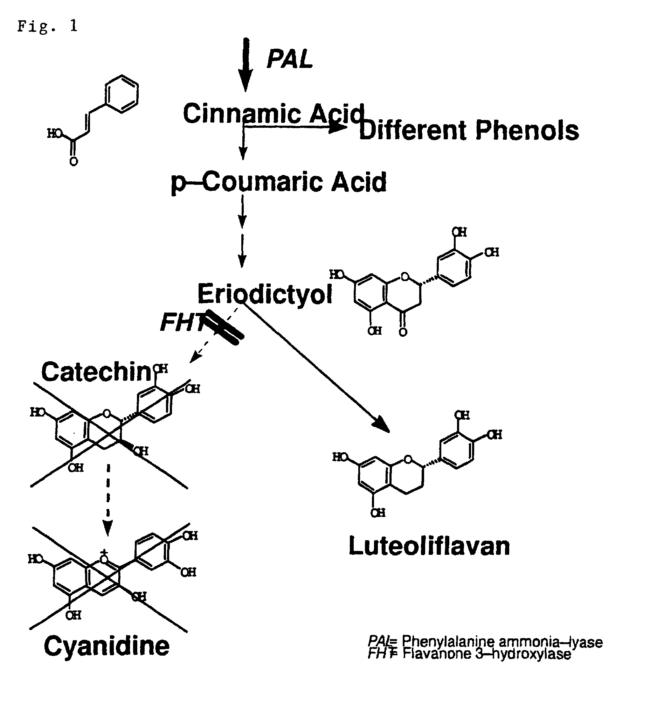

Es kann als gesichert gelten, daß Prohexadion-Ca, Trinexapac-ethyl und andere Acylcyclohexadione 2-Oxoglutarsäure-abhängige Hydroxylasen inhibieren, die im Stoffwechsel phenolischer Substanzen von Bedeutung sind. Dabei handelt es sich primär um Chalconsynthetase (CHS) und um Flavanon-3-hydroxylase (F3H) (W. Heller und G. Forkmann, Biosynthesis, in: The Flavonoids, Harborne, JB (ed.), Chapman and Hall, New York, 1988). Es kann jedoch nicht ausgeschlossen werden, daß Acylcyclohexadione auch weitere, bislang unbekannte, 2-Oxoglutarsäure-abhängige Hydroxylasen hemmen. Es dürfte ferner naheliegend sein, daß ein Mangel an Catechin, Cyanidin oder anderen Endprodukten der Flavonoidsynthese von der Pflanze registriert wird und daß über einen Feedback-Mechanismus die Aktivität des Schlüsselenzyms Phenylalaninammoniumlyase (PAL) erhöht wird. Durch die weiterhin existierende Hemmung von CHS und F3H können diese Flavonoid-Endprodukte jedoch nicht gebildet werden, und es kommt zu einer vermehrten Bildung von Luteoliflavan, Eriodyctiol und anderer Phenole (Abbildung 1).

Aufgabe der Erfindung war es ein kostengünstiges, einfaches Verfahren zu entwickeln, um den Gehalt an Flavonoiden und phenolischen Verbindungen in Pflanzen zu erhöhen und um deren gesundheitsfördernden Eigenschaften zu verbessern.

Die Aufgabe konnte überraschenderweise durch Behandlung der Pflanzen mit den wachstumsregulierenden Verbindungen aus der Gruppe der Acylcyclohexadione(I) 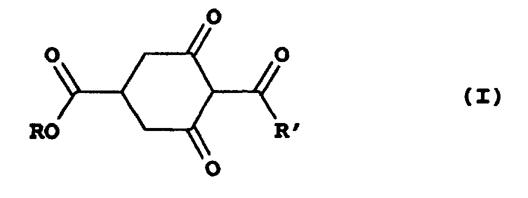 und vor allem mit den Verbindungen Prohexadion-Ca(II) 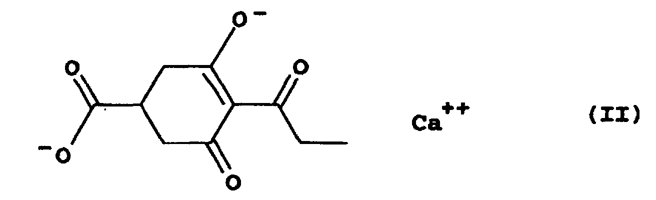 und Trihexapac-ethyl(III) 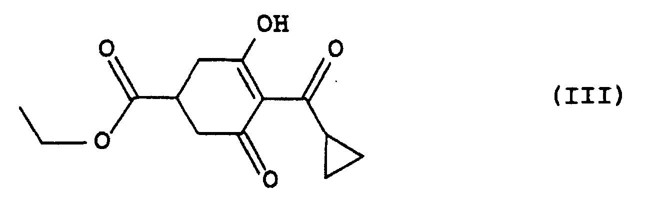 erreicht werden.

Durch die Behandlung der Pflanzen mit den Acylcyclohexadionen der Formel (I), Prohexadion-Ca(II) und Trinexapac-ethyl(III) können die Flavonoide Eriodictyol, Proanthocyanidine, die am C-Atom 3 mit Wasserstoff substituiert sind, z.B. Luteoforol, Luteoliflavan, Apigeniflavan und Tricetiflavan, sowie homogene und heterogene Oligomere und Polymere aus den genannten und strukturell verwandten Substanzen vermehrt gebildet werden.

Erhöhte Konzentrationen der Phenole Hydroxyzimtsäure (p-Cumarsäure, Ferulasäure, Sinapinsäure), Salicylsäure oder Umbelliferon, einschließlich der aus ihnen gebildeten homogenen und heterogenen Oligomere und Polymere können nach Applikation der Verbindungen Acylcyclohexadion der Formel (I), Prohexadion-Ca(II) und Trihexapac-ethyl(III) auf Pflanzen festgestellt werden.

Durch die Behandlung der Pflanzen mit den Acylcyclohexadionen der Formel (I). Prohexadion-Ca(II) und Trinexapac-ethyl(III) wird auch die Konzentration der Glykoside der Flavonoide, der phenolischen Verbindungen, der Chalcone und der Stilbene in den Pflanzen erhöht.

Prohexadion-Ca, Trinexapac-ethyl und verwandte Verbindungen greifen auch in weitere Stoffwechselreaktionen ein, bei denen bislang allenfalls vermutet werden kann, daß 2-Oxoglutarat-abhängige Dioxygenasen beteiligt sind.

Als weitere zusätzlich positive Wirkung bei der Gewinnung von Präparaten aus höheren Pflanzen mit verbesserter heilender, gesundheitsfördernder oder stärkender Wirkung ist zu vermerken, daß sich aufgrund der wachstumsregulierenden Wirkung von Prohexadion-Ca, Trinexapac-ethyl oder verwandter Acylcyclohexandione ein Konzentrierungseffekt der relevanten Inhaltsstoffe im biologischen Material ergibt.

Das erfindungsgemäße Verfahren zur Erhöhung des Gehalts an Flavonoiden und phenolischen Inhaltsstoffen durch Behandlung der Pflanzen mit Verbindungen aus der Gruppe der Acylcyclohexadione der Formel I, speziell Prohexadion-Ca oder Trinexapac-ethyl, kann erfolgreich bei folgenden Pflanzen angewendet werden, wobei auch Pflanzen, die nicht genannt sind, erfolgreich behandelt werden können: Weinrebe, Kirsche, Pflaume, Schlehe, Blaubeere, Erdbeere, Zitrusfrüchte (wie Orange, Grapefruit), Papaya, Rotkohl, Broccoli, Rosenkohl, Grünkohl, Karotte, Petersilie, Sellerie, Zwiebeln, Knoblauch, Tee, Kaffee, Kakao, Maté, Hopfen, Soja, Raps, Hafer, Weizen, Roggen, Aronia melanocarpa, Ginkgo biloba.

Pflanzen, die mit Verbindungen aus der Gruppe der Acylcyclohexadione, speziell Prohexadion-Ca oder Trihexapac-ethyl, behandelt wurden zur Steigerung des Gehaltes an Flavonoiden und phenolischen Verbindungen, oder von Teilen dieser Pflanzen oder aus ihnen hergestellte Produkte (Säfte, Tees, Extrakte, Fermentationsprodukte und -rückstände) können zur Herstellung von heilenden, gesundheitsfördernden oder stärkenden Mitteln für Mensch und Tier sowie von Kosmetika verwendet werden.

Aus den erfindungsgemäß behandelten Pflanzen können auch Mittel hergestellt werden, die dadurch gekennzeichnet sind, daß Trauben von roten Weinreben gewonnen und verarbeitet werden, deren Anthocyanbildung durch Behandlung mit Acylcyclohexadionen wie Prohexadion-Ca oder Trinexapac-ethyl ganz oder teilweise unterbunden worden ist und die sich daher durch einen qualitativ und quantitativ erhöhten Gehalt an Flavonoiden und anderen phenolischen Inhaltsstoffen auszeichnen.

Es wurde nun überraschend gefunden, daß unter dem Einfluß von mit Acylcyclohexadionen der Formel I, Prohexadion-Ca oder Trihexapac-ethyl behandelten Pflanzen oder von Teilen dieser Pflanzen oder aus ihnen hergestellter Produkte (Tees, Extrakte, Fermentationsprodukte, Säfte etc.)
(I) die Antioxidative Kapazität *in vitro* (Electron Spin Resonance (ESR), LDL-Oxidation, Total Antioxidant Capacity, NO-Scavenging) verbessert wird;
(2) eine modulierende Wirkung auf Enzyme, vor allem Enzyme der Signaltransduktion (Proteinkinase C, Tyrosin-Proteinkinase, Phosphatidylinositol-3-Kinase) auftritt;
(3) eine Modulation redoxsensitiver Transkriptionsfaktoren (NF-kB, AP-1) in Endothelzellen, Lymphocyten und glatten Muskelzellen induziert wird;
(4) die Regulation der Genexpression von Targetgenen involviert in die Pathogenese inflammatorischer Erkrankungen (Cytokine IL-1 und IL-8, *macrophage chemoattractant protein* 1 (MCP-1), Adhesionsfaktoren ICAM-1 und VCAM-1) moduliert wird;
(5) eine antiaggregatorische Wirkung induziert wird;
(6) die Cholesterinsynthese in Hepatocyten gehemmt wird;
(7) antiproliferative/antineoplastische Effekte bestehen.

### Beispiel 1

### Steigerung des Gehaltes an Eriodictyol und Luteoliflavan an jungen Apfelblättern nach Behandlung mit Prohexadion-Ca

Apfelpflanzen der Sorte "Weirouge" wurden unter Klimakammer-Bedingungen kultiviert und mit 250 ppm an Prohexadion-Ca (formuliert als BAS 125 10 W = 10 %iges benetzbares Granulat) bis tropfnaß behandelt. Zu verschiedenen Zeitpunkten nach der Behandlung wurde von den einzelnen Trieben das jeweils jüngste vollentwickelte Blatt geerntet. Die gefriergetrockneten und gemörserten Blätter wurden mit Methanol extrahiert. Aus dem konzentrierten Extrakt wurden Flavonoide und verwandte Verbindungen durch HPLC analysiert. Die Auftrennung erfolgte dabei an Hypersil ODS (3 µm Partikelgröße) auf einer Säule von 250 x 4 mm. Die Elution erfolgte bei einer Fließrate von 0,5 ml pro Minute, wobei Mischungen aus Ameisensäure (5 %ig in Wasser) und Methanol, schrittweise im Verhältnis von 95 : 5 bis 10 : 90 (v/v) gesteigert, verwendet wurden. Phenolische Säuren und Flavonole wurden bei 280 nm detektiert. Flavan-3-ole wurden nach Nachsäulenderivatisierung mit p-Dimethylaminozimtaldehyd bei 640 nm bestimmt. Methodische Details bei Treutter et al. (1994), Journal of Chromatography A 667, 290 - 297.

Das Ergebnis ist in folgender Tabelle dargestellt:

Blätter, die mit Prohexadion-Ca behandelt wurden zeigen eine deutliche Erhöhung der Eriodictyol-Konzentration nach 12 bzw. 21 Tagen.

| Behandlung | Eriodictyol [g/kg Trockenmasse) | | Luteoliflavan [g/kg Trockenmasse] | |
|---|---|---|---|---|
| | 12 Tage n. Beh. | 21 Tage n. Beh. | 12 Tage n. Beh. | 21 Tage n. Beh. |
| Kontrolle | 0 | 1 | 0 | 70 |
| 250 ppm Prohexadion-Ca | 17 | 27 | 0 | 34 |

### Beispiel 2

Herstellung von Probenmaterialien aus behandelten und unbehandelten Dornfelder-Trauben

Rebstöcke der Sorte "Dornfelder" wurden zweimal zu verschiedenen Zeitpunkten mit der Formulierung BAS 125 10W, enthaltend Prohexadion-Ca behandelt. Je Behandlung wurden 1000 g Prohexadion-Ca in 1000 1 Spritzbrühe pro ha appliziert.

Die 1. Ausbringung erfolgte im Entwicklungstadium 73 noch vor Beginn der Beerenausfärbung, die 2. Ausbringung 10 Tage später.

Bei der Ernte wiesen die unbehandelten und die behandelten Trauben einer ähnlichen Reifegrad auf. Kontrolle unbehandelt: 69°C Oe; 7,3 g/l Säurebehandelt: 67° Oe; 7,4 g/l Säure.

Die behandelten Trauben waren geringer pigmentiert. Geschmacklich war kein Unterschied feststellbar.

Der Weinausbau erfolgte nach gängigen Methoden als Rotwein, d.h. zur besseren Pigmentextraktion stand der Most längere Zeit auf der Maische.

Nach Gefriertrocknung des trübungsfreien Weins wurden aus 100 ml unbehandeltem Wein ca. 2,5 g sirupartiger Rückstand und aus dem mit Prohexadion-Ca behandelten Rebstöcken gewonnenen Wein ca. 2,1 g sirupartiger Rückstand gewonnen.

### Beispiel 3

### Hemmung der Cholesterinbiosynthese in Kulturen primärer Rattenhepatocyten durch Prohexadion-Ca behandelten Dornfelder Wein

### Herstellung der Stammlösungen

Vom Lyophilisat der unbehandelten und behandelten Dornfelder Weine wurde eine Menge zwischen 10 und 20 mg exakt abgewogen und mit soviel DMSO versetzt, daß eine Stammlösung von 10 mM Gesamtflavonoide entstand. Von diesen Stammlösungen wurden unmittelbar vor Testbeginn Verdünnungen im Kulturmedium hergestellt. Die Verdünnungen erfolgten in 10er Schritten zwischen 10⁻⁴ und 10⁻⁸ M.

### Herstellung der Hepatocytenkulturen

Primäre Hepatocyten wurden aus den Lebern von männlichen Spraque-Dawley Ratten (240-290 g) mittels Collagenase-Perfusion gewonnen (Gebhardt et al., Arzneimittel-Forschung/Drug Res. 41: 800 -804 (1991) 1990). Die Kultivierung erfolgte in Collagen-beschichteten Petrischalen (6-well Plates, Greiner, Nürtingen) mit einer Zeltdichte von 125.000 Zellen/cm² in Williams Medium E mit 10 % Kälberserum. Nähere Angaben insbesondere zum Kulturmedium finden sich bei Gebhardt et al., Cell Biol. Toxicol. 6: 369 - 372 (1990) und Mewes et al., Cancer Res. 53: 5135 - 5142 (1993). Die Kulturen wurden nach 2 h auf serum-freies Medium mit Zusatz von 0,1 µM Insulin gewechselt. Sie wurden nach weiteren 20 h für die Versuche eingesetzt. Die Testsubstanzen wurden in je drei unabhängigen Kulturen von 2-3 Ratten getestet.

### Inkubation der Leberzellkulturen mit den Testsubstanzen

Für den Nachweis einer Beeinflussung der Cholesterin-Biosynthese durch die Testsubstanzen wurden die Hepatocytenkuturen insgesamt für 22 h gehalten. Anschließend wurde mit serum-freiem Williams Medium E unter Zusatz von ¹⁴C-Acetat (nur Tracermengen) für 2 h mit den Testsubstanzen in den angegebenen Konzentrationen inkubiert. Bei jeder Testserie wurde eine Kontrolle mitgeführt. Die Methodik ist bei Gebhardt (1991) und Gebhardt, Lipids 28: 613 -619 (1993) detailliert beschrieben. Die Tracermengen von ¹⁴C-Acetat tauschen schnell mit dem intrazellulären Acetyl-CoA Pool aus und ermöglichen deshalb eine störungsfreie Bestimmung des Einbaus von ¹⁹C-Acetat in die Sterolfraktion, die zu >90 % aus Cholesterin besteht (Gebhardt, 1993).

### Analytik zur Beeinflussung der Cholesterin-Biosynthese

Der Einbau von ¹⁴C-Acetat in die Sterolfraktion (nicht-verseifbare Lipide) wurde nach Gebhardt (1991) gemessen. Bei der verwendeten Extraktion mittels Extrelut®-Säulen (Merck, Darmstadt) wird das ¹⁴C-Acetat (und daraus in geringer Menge entstehende andere niedermolekulare Metabolite) zu mehr als 95 % abgetrennt. Dieser

Test kann vergleichende Angaben über die relative Syntheserate von Cholesterin und Vorläufer-Sterolen unter dem Einfluss von Testsubstanzen machen (Gebhardt, 1993).

Visuelle und mikrobielle Überprüfung der Qualität der Hepatocytenkulturen

Alle verwendeten Kulturen wurden vor und nach der Testinkubation visuell am Mikroskop auf Kontamination mit Mikroorganismen und auf die Integrität der Zellmonolayer überprüft. Bei keiner der Proben wurde eine erkennbare Veränderung der Zellmorphologie (insbesondere bei den höheren Konzentrationen) beobachtet. Dies schließt eine Beeinflussung der Testergebnisse durch zytotoxische Wirkungen der Testsubstanzen weitgehend aus.

Die bei allen Kulturen routinemäßig durchgeführten Sterilitätstests ergaben keinerlei Hinweise auf eine Kontamination mit Mikroorganismen.

### Ergebnisse

Der unbehandelte Dornfelder Wein zeigte keinerlei Wirkungen auf die Cholesterinbiosynthese. Dagegen wurde die Cholesterinsynthese durch Proben von mit Prohexadion-Ca behandelten Rebstöcken hergestelltem Wein signifikant inhibiert. Bei einer Konzentration von 10⁻⁵ M betrug die Hemmwirkung ca. 60 % und bei 10⁻⁴ M annähernd 100 %.

### Beispiel 4

Effekt von Prohexadion-Ca-behandeltem Weinextrakt (P-Ca) auf die Destruktion von Tumorzellen

Konfluente murine Leukämiezellen (RAW 264.7) und normale Makrophagen aus dem Peritoneum von Ratten wurden in mit foetalem Kälberserum supplementiertem DMEM-Medium kultiviert. Extrakte von unbehandeltem und mit Prohexadion-Ca behandeltem Wein wurden bis zu einer Dosierung von 200 µg/mL dem Kulturmedium zugesetzt. In Parallelexperimenten wurden 10, 25 und 50 µg/mL Weinextrakt gemeinsam mit 100 µM H₂O₂ inkubiert.

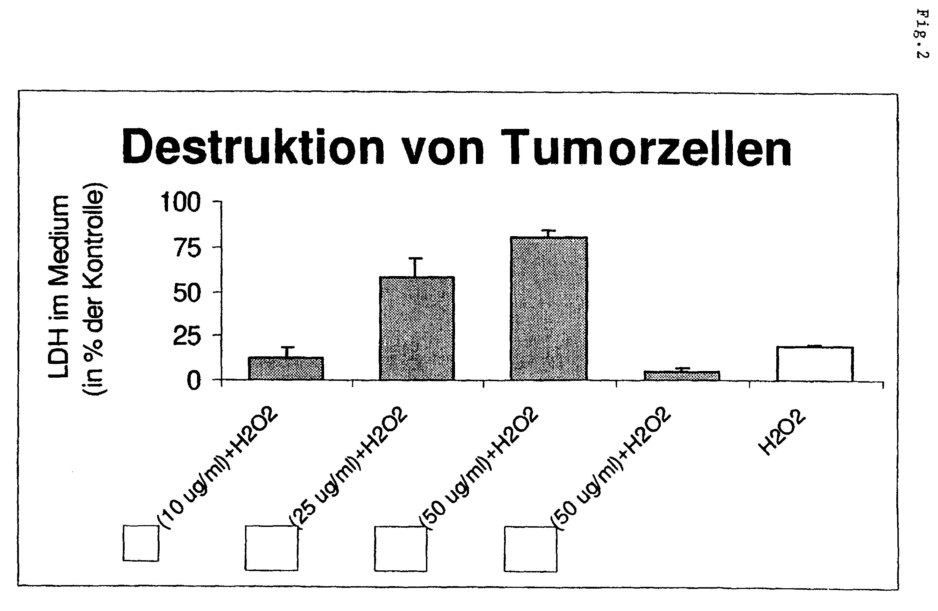

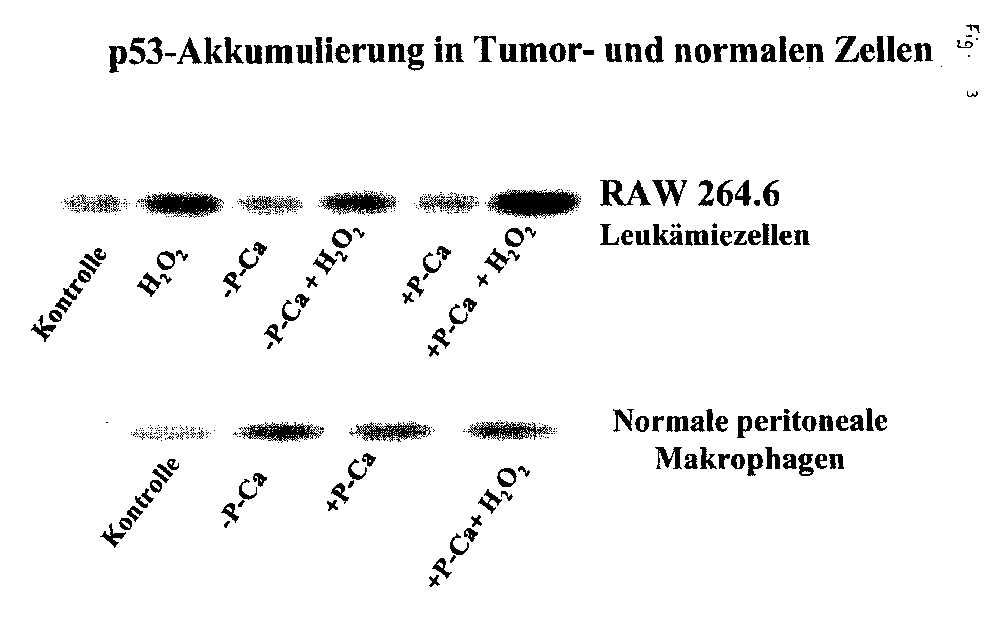

Weinextrakt von mit Prohexadion-Ca-behandeltem Wein hatte *per se* bis zu einer Dosierung von 200 µg/mL keinen cytotoxischen Effekt auf die untersuchten Zellkulturen. Jedoch verstärkte Prohexadion-Ca-behandelter Extrakt nach Zugabe von H₂O₂ dosisabhängig den Zelltod der Tumorzellen (RAW 264.7). Dies wird in Abbildung 2 durch die Zunahme des cytosolischen Enzyms Lactatdehydrogenase (LDH) im Kulturmedium dokumentiert. Keine Erhöhung der cytotoxischen Wirkung von H₂O₂ trat bei den nicht-transformierten Makrophagen auf. Bei der Tumorzellinie trat eine Akkumulation des Proteins vom Tumorsuppressorgen p53 im Cytoplasma auf, siehe Abbildung 3.

Prohexadion-behandelter Weinextrakt erhöht die H₂O₂-induzierte Cytotoxizität von Leukämiezellen, ist aber nicht bei normalen Makrophagen wirksam. Dieser für Tumorzellen spezifische Effekt tritt auch bei Cytostatika auf, die über vermehrten oxidativen Stress wirken (z.B. Anthracycline). Der Mechanismus von Prohexadion-Ca-behandeltem Weinextrakt ist p53-abhängig.

### Beispiel 5

### Effekt von Prohexadion-Ca-behandeltem Weinextrakt auf die NF-κB-Aktivierung in Endothelzellen

Die Untersuchung wurde in Cokulturen aus Makrophagen (RAW 264.7) und Endothelzellen (ECV 304) durchgeführt. Dem Kulturmedium der Endothelzellen wurde humanes LDL (Lipoproteine niedriger Dichte) und ruhende bzw. Interferon-γ (IFN-γ)-aktivierte (10 U/mL) Makrophagen zugegeben. Nach 16 h Inkubation wurde die nukleäre Proteinfraktion abgetrennt und in elektrophoretischen Mobility Shift Assays die DNA-Bindung (Aktivierung) des redoxsensitiven Transkiptionsfaktors NF-κB bestimmt.

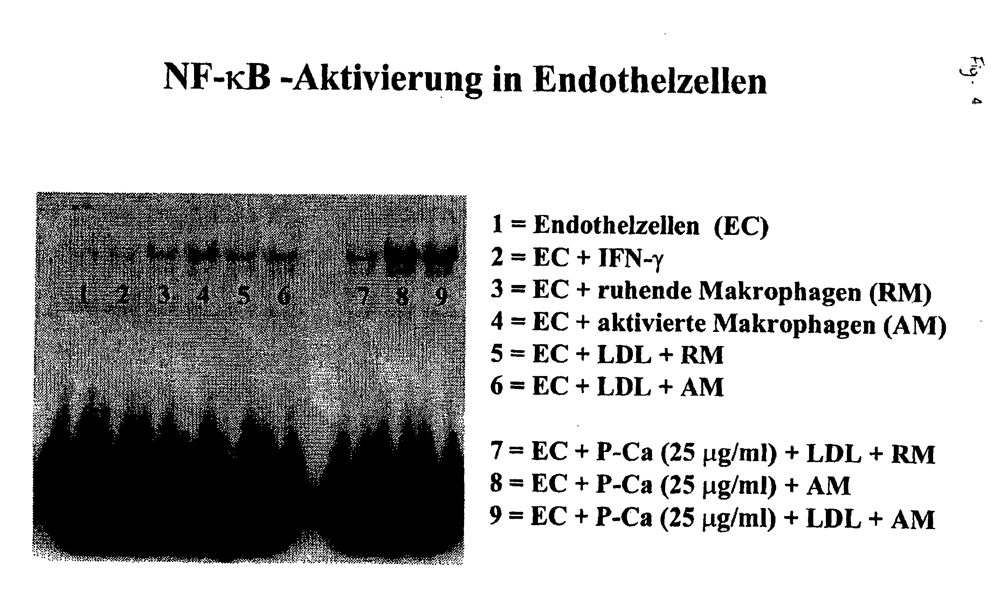

Der basale Gehalt in den ruhenden Endothelzellen war typischerweise gering, siehe Abbildung 4. Die Zugabe von LDL hatte eine Aktivierung des NF-κB zur Folge, die bei aktivierten Makrophagen höher war als bei den ruhenden. Dies entspricht einer physiologischen Oxidation der LDL bei der Atherogenese. Durch die Inkubation mit Prohexadion-Ca-behandeltem Weinextrakt war die NF-κB-Aktivierung in allen Fällen erhöht.

Das verwendete Zellkulturmodell ist sehr gut geeignet, die pathophysiologischen/ inflammatorischen Bedingungen in der frühen Phase der Atherosklerose zu beschreiben. Durch den Prohexadion-Ca-behandelten Weinextrakt wird die NF-κB-Aktivierung verstärkt. Dies kommt der Wirkung eines Biological Response Modifiers gleich; d.h. die Zellantwort auf ein pathophysiologisches Signal wird im positiven Sinne verstärkt.

## Patentansprüche

1. Verfahren zur Erhöhung und qualitativen Veränderung des Gehalts an Flavonoiden und phenolischen Inhaltsstoffen in Weinreben, Kirsche, Pflaume, Schlehe, Blaubeere, Erdbeere, Zitrusfrüchten, Papaya, Rotkohl, Broccoli, Rosenkohl, Grünkohl, Karotte, Petersilie, Sellerie, Zwiebeln, Knoblauch, Tee, Kaffee, Kakao, Maté, Hopfen, Soja, Raps, Hafer, Weizen, Roggen, *Aronia melanocarpa* oder *Ginkgo biloba,* **dadurch gekennzeichnet, daß** die Pflanzen mit einem Acylcyclohexadion der Formel I 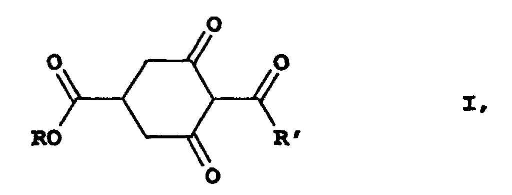 wobei R für Wasserstoff oder C₁-C₆-Alkyl und R' für C₁-C₆-Alkyl oder C₃-C₆-Cycloalkyl stehen, oder einem geeigneten Salz von I, behandelt werden.

2. Verfahren nach Anspruch 1, wobei die Pflanzen mit einem Acylcyclohexadion der Formel II und/oder Formel III 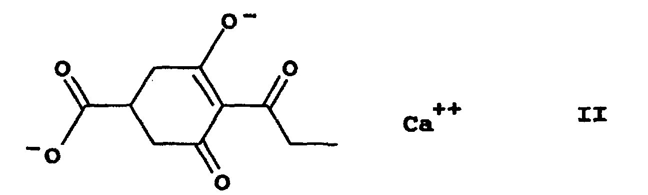 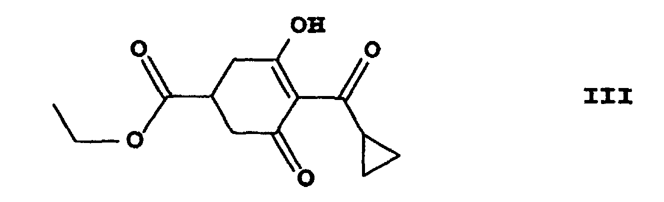 behandelt werden.

3. Verfahren nach Anspruch 1 oder 2, **dadurch gekennzeichnet, daß** der Gehalt an Flavonoiden und phenolischen Inhaltsstoffen in Weinreben erhöht und qualitativ verändert wird.

4. Verfahren nach Anspruch 1, 2 oder 3, **dadurch gekennzeichnet, daß** der Gehalt an Flavonoiden mit unsubstituiertem C-Atom in 3-Position, sowie deren Oligomeren und Polymeren erhöht wird.

5. Verwendung von Weinrebe, Kirsche, Pflaume, Schlehe, Blaubeere, Erdbeere, Zitrusfrüchten, Papaya, Rotkohl, Broccoli, Rosenkohl, Grünkohl, Karotte, Petersilie, Sellerie, Zwiebeln, Knoblauch, Tee, Kaffee, Kakao, Maté, Hopfen, Soja, Raps, Hafer, Weizen, Roggen, *Aronia melanocarpa* oder *Ginkgo biloba,* die mit einem Acylcyclohexadion gemäß Anspruch 1 oder 2 behandelt wurden, von Teilen dieser Pflanzen oder aus ihnen hergestellte Produkte (Säfte, Tees, Extrakte, Fermentationsprodukte und -rückstände) zur Herstellung von heilenden, gesundheitsfördernden oder stärkenden Mitteln für Mensch und Tier sowie von Kosmetika.

6. Extrakte, Säfte, Weine oder Preßrückstände mit erhöhtem und qualitativ verändertem Gehalt an Flavonoiden und anderen phenolischen Inhaltsstoffen, erhältlich aus Trauben von Weinpflanzen einer roten Sorte, wobei die Weinpflanzen vorher mit mindestens einem Acylcyclohexadion der Formel I, II oder III gemäß Anspruch 1 oder 2 behandelt wurden.

## Claims

1. A method of increasing and qualitatively modifying the content of flavonoids and phenolic constituents in grapevines, cherries, plums, sloes, blueberries, strawberries, citrus fruit, pawpaw, red cabbage, broccoli, Brussels sprouts, kale, carrots, parsley, celery/celeriac, onions, garlic, tea, coffee, cacao, maté, hops, soya, oilseed rape, oats, wheat, rye, *Aronia melanocarpa* or *Ginkgo biloba,* which comprises treating the plants with an acylcyclohexanedione of the formula I 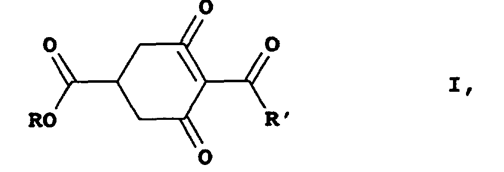 where R is hydrogen or C₁-C₆-alkyl and R' is C₁-C₆-alkyl or C₃-C₆-cycloalkyl, or with a suitable salt of I.

2. A method as claimed in claim 1, wherein the plants are treated with an acylcyclohexanedione of the formula II and/or the formula III

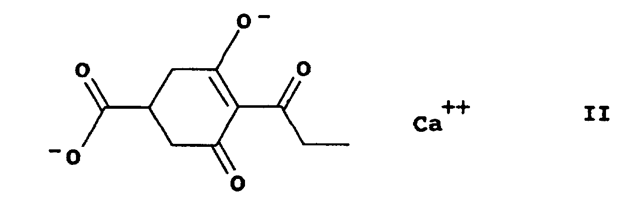

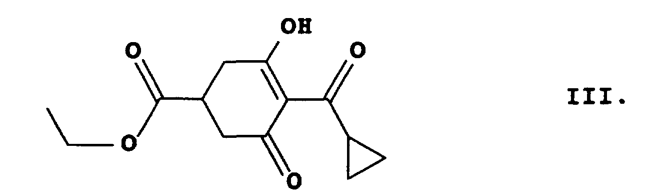

3. A method as claimed in claim 1 or 2, wherein the content of flavonoids and phenolic constituents of grapevines is increased and qualitatively modified.

4. A method as claimed in any of claims 1, 2 or 3, wherein the content of flavonoids with an unsubstituted C atom in the 3-position, and of the oligomers and polymers of these flavonoids, is increased.

5. The use of grapevines, cherries, plums, sloes, blueberries, strawberries, citrus fruit, pawpaw, red cabbage, broccoli, Brussels sprouts, kale, carrots, parsley, celery/celeriac, onions, garlic, tea, coffee, cacao, maté, hops, soya, oilseed rape, oats, wheat, rye, *Aronia melanocarpa* or *Ginkgo biloba,* which have been treated with an acylcyclohexanedione as set forth in claim 1 or 2, of parts of these plants or of products prepared with these plants (juices, teas, extracts, fermentation products and fermentation residues) for the preparation of curative compositions, health-promoting compositions or tonics for humans and animals, and of cosmetics.

6. An extract, juice, wine or press cake with an increased qualtitatively modified content of flavonoids and other phenolic constituents, obtainable from grapes of a red grapevine variety, the grapevine plant previously having been treated with at least one acylcyclohexanedione of the formula I, II or III as set forth in claim 1 or 2.

## Revendications

1. Procédé pour l'augmentation et la modification qualitative de la teneur en flavonoïdes et en composants phénoliques dans la vigne, la cerise, la prune, la prunelle, la myrtille, la fraise, les agrumes, la papaye, le chou rouge, le brocoli, le chou de Bruxelles, le chou vert, la carotte, le persil, le céleri, les oignons, l'ail, le thé, le café, le cacao, le maté, le houblon, le soja, le colza, l'avoine, le blé, le seigle, *Aronia melanocarpa* ou *Ginkgo biloba,* **caractérisé par le fait que** les plantes sont traitées avec une acylcyclohexadione de formule I 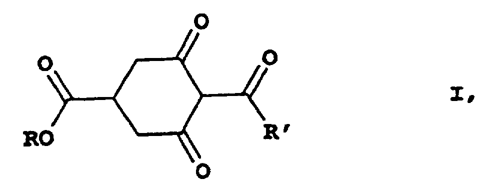 où R désigne l'hydrogène ou un alkyle en C₁-C₆ et R' désigne un alkyle en C₁-C₆ ou un cycloalkyle en C₃-C₆, ou un sel approprié de I.

2. Procédé selon la revendication 1, dans lequel les plantes sont traitées avec une acylcyclohexadione de formule II et/ou de formule III

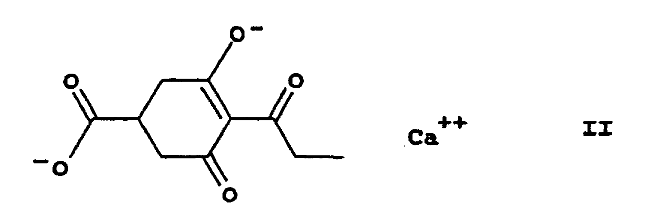

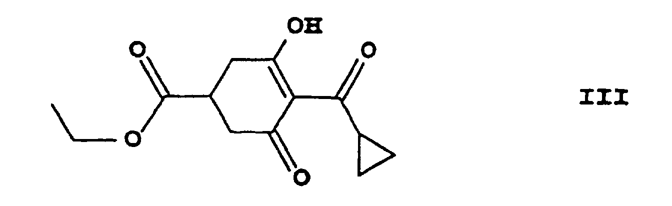

3. Procédé selon la revendication 1 ou 2, **caractérisé par le fait que** la teneur en flavonoïdes et en composants phénoliques dans la vigne est augmentée et modifiée qualitativement.

4. Procédé selon la revendication 1, 2 ou 3, **caractérisé par le fait que** la teneur en flavonoïdes est augmentée avec l'atome C non-substitué en poisition 3, ainsi que leurs oligomères et polymères.

5. Utilisation de vigne, cerise, prune, prunelle, myrtille, fraise, agrumes, papaye, chou rouge, brocoli, chou de Bruxelles, chou vert, carotte, persil, céleri, oignons, ail, thé, café, cacao, maté, houblon, soja, colza, avoine, blé, seigle, *Aronia melanocarpa* ou *Ginkgo biloba,* qui ont été traités avec une acylcyclohexadione selon la revendication 1 ou 2, de parties de ces plantes ou de produits préparés à partir d'elles (jus, infusions, extraits, produits et résidus de fermentation) pour la préparation de produits réparateurs, favorisant la santé ou la renforçant pour les humains et les animaux, ainsi que de cosmétiques.

6. Extraits, jus, vins ou résidus de pressage ayant une teneur accrue et modifiée qualitativement en flavonoïdes et en autres composants phénoliques, pouvant être obtenus à partir de raisins de vigne d'une variété rouge, tandis que les vignes ont au préalable été traitées avec au moins une acylcyclohexadione de formule I, II ou III selon la revendication 1 ou 2.
